# EUROPEAN PATENT APPLICATION

(11) **EP 4 487 699 A1**
(43) Date of publication of application: **08.01.2025**
(21) Application number: 23763509.9
(22) Date of filing: 01.03.2023
(51) Int. Cl.: A23L 33/13, A61K 31/7064, A61K 31/7068, A61K 31/7072, A61P 3/00, A61P 3/04, A61P 15/00, A61P 25/00, A61P 25/22, A61P 25/24, A61P 25/28, A61P 43/00, C07K 14/575

(54) **BDNF PRODUCTION PROMOTER**

(30) Priority: 01.03.2022 JP 2022030759
(71) Applicant: YAMASA CORPORATION, Choshi-shi, Chiba 288-0056 (JP)
(72) Inventor: NAKAGAWARA,Kosuke, Choshi-shi, Chiba 288-0056 (JP); ISHIGE,Kazuya, Choshi-shi, Chiba 288-0056 (JP)
(74) Representative: Maiwald GmbH
(86) International application number: PCT/JP2023/007663
(87) International publication number: WO 2023/167252

(57) **Abstract**

A BDNF production promoter includes at least one pyrimidine nucleotide or a precursor thereof as an active ingredient.

## Description

### [Technical Field]

The present invention relates to a BDNF production promoter.

### [Background Art]

Aging and stress cause deterioration or abnormalities of brain functions, such as cognitive decline and memory loss, depression, and insomnia, which in turn reduce quality of life (QOL). In today's stressful and aging society, neuropsychiatric disorders such as depression and dementia are becoming major problems, and countermeasures are being required.

BDNF (Brain-derived neurotrophic factor) is a growth factor having functions such as maintenance of neuronal survival, promotion of neurite outgrowth, regulation of synaptic plasticity, and protection from brain damage, and is an important molecule for brain functions such as learning and memory (Non-Patent Literature 1).

BDNF is also found in muscle and is regarded as a type of myokine (a hormone released by muscle) because it increases with exercise. BDNF is produced not only in skeletal muscle but also in various tissues in the body, including the nervous system. BDNF is abundant in platelets and circulates throughout the body as well as in the brain. BDNF is known to have functions such as promotion of glucose and lipid metabolism, control of appetite and eating behavior, maintenance of erectile ability (non-Patent Literature 1), and maturation of egg (Non-Patent Literature 2), in tissues throughout the body as well as in the brain.

It is known that a moderate increase in BDNF *in vivo* has effects such as improvement of memory, prevention or improvement of depression, improvement of glucose and lipid metabolism, and suppression of appetite (Non-Patent Literature 1).

On the other hand, it is known that BDNF levels are decreased by aging, the onset of depression and Alzheimer's disease, and exposure to chronic stress (Non-Patent Literature 3).

Thus, an increase in BDNF is expected to produce various effects such as improved cognitive function and/or memory, prevention or improvement of depression, prevention or improvement of neurodegenerative diseases, reduction of anxiety, improvement of energy, improvement of glucose and lipid metabolism, anti-obesity effects, and improvement of reproductive function.

Therefore, research to increase BDNF has been actively conducted, and recently, BDNF production promoters that use food materials and the like have been reported. For example, Patent Literature 1 proposes a BDNF secretion stimulating agent including an oligopeptide mixture containing a dipeptide or tripeptide having lysine and/or arginine as an active ingredient. Also, Patent Literature 2 proposes a BDNF production promoter containing extracts of rosemary, lemon, lavender, marjoram, cypress, and orange as active ingredients.

Cytidylic acid and uridylic acid are a kind of nucleotides, and are substances widely contained in or added to living bodies or foods, and are extremely safe and ideal materials for addition to foods.

Patent Literature 3 reports an anti-psychosocial stress agent containing UMP or uridine. Patent Literature 4 reports an agent for improving impaired learning and memory ability comprising UMP, GMP, uridine or guanosine. Patent Literature 5 reports a lipid metabolism regulator characterized by containing UMP and/or uridine as active ingredients.

However, Patent Literatures 3 to 5 do not conduct any study for BDNF, and whether nucleotides such as cytidylic acid and uridylic acid or nucleosides such as cytidine and uridine actually have an effect of promoting the BDNF production is not conventionally examined and is not revealed at all.

### [Citation List]

### [Patent Literatures]

[PTL 1]
   WO 2017/150327 A1
[PTL 2]
   Japanese Patent Application Publication No. 2021-63018 A
[PTL3]
   WO 2008/001495 A1
[PTL 4]
   Japanese Patent Application Publication No. 2001-233776 A
[PTL 5]
   Japanese Patent Application Publication No. 2000-319185 A

### [Non-Patent Literature]

[NON-PTL 1]
   Koji Yanamoto et al., "Brain-derived neurotrophic factor (BDNF), a secretory protein that enhances brain function", The Medical Journal of Rakuwakai H.C.S, Vol. 28: 7-24, 2017
[NON-PTL 2]
   K. Kawamura et al., "Ovarian brain-derived neurotrophic factor (BDNF) promotes the development of oocytes into preimplantation embryos", Proc Natl Acad Sci USA: 9206-9211, 2005
[NON-PTL 3]
   M. Miranda et al., "Brain-Derived Neurotrophic Factor: A Key Molecule for Memory in the Healthy and the Pathological Brain", Front. Cell. Neurosci. 2019.00363

### [Summary of Invention]

### [Technical Problem]

An object of the present invention is to provide a novel and highly safe BDNF production promoter that exhibits a BDNF production promoting effect when it is used as a food, feed, drug, quasi-drug, or the like.

### [Solution to Problem]

As a result of intensive studies to achieve the above object, the present inventors have first found that pyrimidine nucleotides or precursors thereof have a clear effect of promoting the BDNF production, and they have completed the present invention.

In other words, the present invention is a BDNF production promoter comprising at least one pyrimidine nucleotide or a precursor thereof as an active ingredient.

### [Advantageous Effects of Invention]

The present invention provides a novel and highly safe BDNF production promoter. The present invention would lead to a novel means for improving the quality of life of patients suffering from diseases caused by deficiency and/or decrease in BDNF, or elderly persons.

### [Brief Description of Drawings]

[FIG. 1]

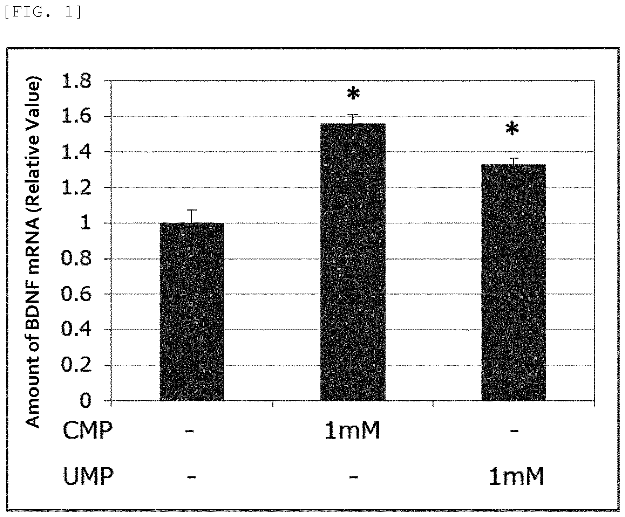

   FIG. 1 shows an effect of increasing BDNF mRNA by treatment with cytidylic acid or uridylic acid on myotube cells derived from C2C12 cells in Example 1; n = 3, error bars: standard error, *: p < 0.05.
[FIG. 2]

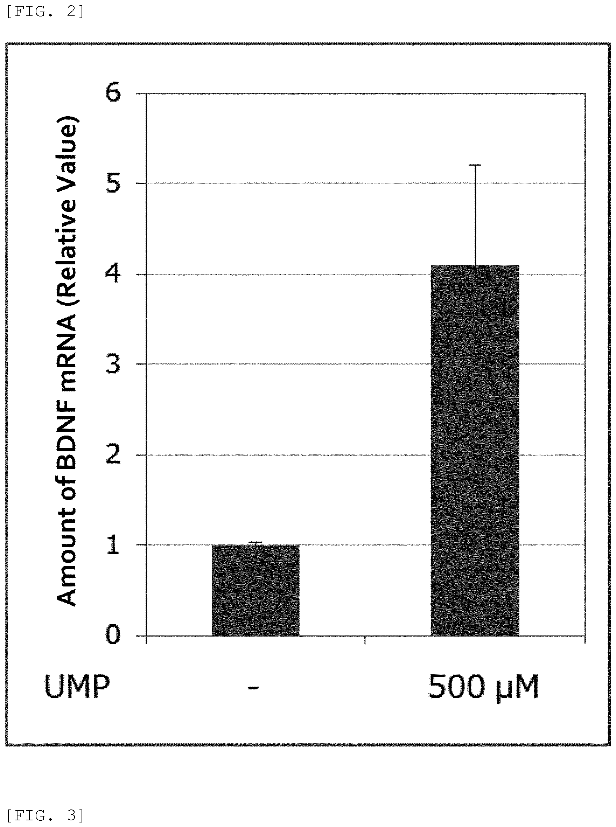

   FIG. 2 shows an effect of increasing BDNF mRNA by treatment with uridylic acid on PC-12 cells in Example 2; n = 3, error bars mean standard error.
[FIG. 3]
   FIG. 3 shows an effect of increasing BDNF mRNA by treatment with uridine on PC-12 cells in Example 3; n = 3, error bars: standard error, *: p < 0.05.
[FIG. 4]

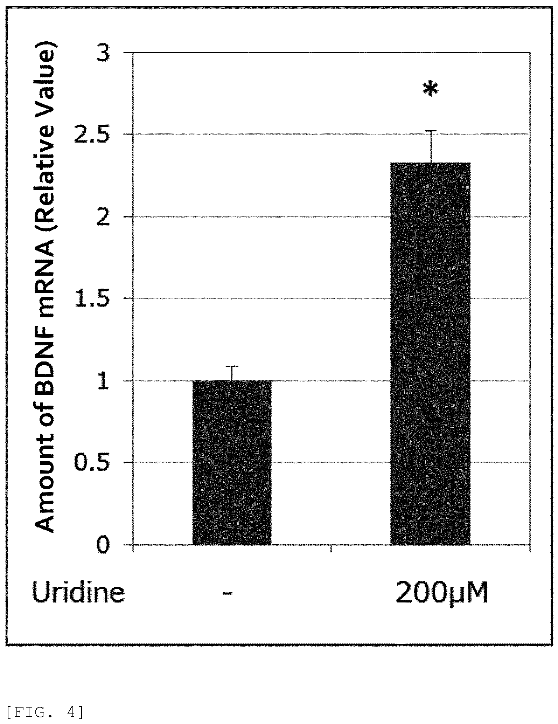

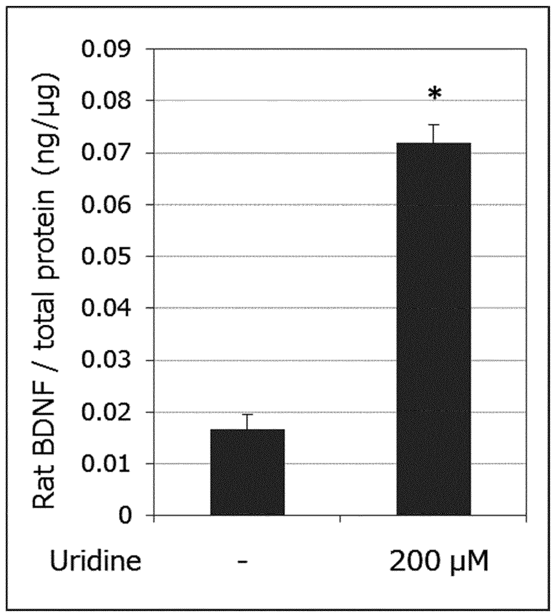

   FIG. 4 shows an effect of increasing a BDNF protein by treatment with uridine on PC-12 cells in Example 4; n = 3, error bars: standard error, *: p < 0.05.

### [Description of Embodiments]

The present invention relates to a BDNF production promoter containing at least one pyrimidine nucleotide or a precursor thereof as an active ingredient.

The BDNF production promoter according to the present invention contains at least one pyrimidine nucleotide or a precursor thereof as an active ingredient.

As used herein, the pyrimidine nucleotide means cytidylic acid and/or uridylic acid.

The cytidylic acid (cytidine monophosphate, cytidine 5'-phosphate, CMP) is a compound represented by CAS Registry Number 63-37-6. When cytidylic acid is mentioned herein, salts of cytidylic acid are also included.

When a mass of cytidylic acid is described herein, it represents a mass when converted to cytidylic acid disodium salt (CMP,2Na). When a concentration (%) of a cytidylic acid solution is mentioned herein, a mass converted to CMP,2Na is used as the mass of cytidylic acid. If a salt other than the disodium salt is selected, or if it is a free acid that does not form a salt, it is a mass when converted to CMP,2Na, based on an amount of substance of the cytidylic acid.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to uridylic acid disodium salt (UMP,2Na). When a concentration (%) of a uridylic acid solution is mentioned herein, a mass converted to UMP,2Na is used as the mass of uridylic acid. If a salt other than the disodium salt is selected, or if it is a free acid that does not form a salt, it is a mass when converted to UMP,2Na, based on an amount of substance of the uridylic acid.

As used herein, a pyrimidine nucleotide precursor means a compound that can be metabolized to the pyrimidine nucleotide, i.e., cytidylic acid and/or uridylic acid. Whether a compound is included in the pyrimidine nucleotide precursor is determined by the presence or absence of knowledge that the compound is converted to the pyrimidine nucleotide. Specifically, cytidine diphosphate, cytidine triphosphate, uridine diphosphate, and uridine triphosphate, which are known to be degraded to cytidylic acid and/or uridylic acid by the action of ectonucleotidases and like (Isao Matsuoka, "Ectonucleotidases in Nervous System", Japanese Journal of Clinical Chemistry 33: 11-18, 2004), and cytidine, cytosine, uridine, and uracil, which are known to be phosphorylated to cytidylic acid and/or uridylic acid by the action of kinases (A Orengo, "Regulation of enzymic activity by metabolites. I. Uridine-cytidine kinase of Novikoff ascites rat tumor", J Biol Chem. 1969 Apr 25; 244(8): 2204-2209.) are exemplified as pyrimidine nucleotide precursors as used herein.

The present invention is a BDNF production promoter containing a pyrimidine nucleotide or a precursor thereof as an active ingredient.

Examples of the pyrimidine nucleotides or precursors thereof according to the present invention include, as described above, cytidine, cytosine, cytidylic acid, cytidine diphosphate, cytidine triphosphate, uridine, uracil, uridylic acid, uridine diphosphate, and uridine triphosphate. Among them, cytidine, cytosine, cytidylic acid, cytidine diphosphate, uridine, uracil, uridylic acid and uridine diphosphate are preferred, and cytidylic acid, uridylic acid, cytidine, and uridine are more preferred.

The concept of cytidylic acid as used herein includes salts as described above. The salts of cytidylic acid include alkali metal salts such as sodium and potassium salts; alkaline earth metal salts such as calcium, magnesium and barium salts; basic amino acid salts such as arginine and lysine; ammonium salts such as ammonium and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, alkali metal salts such as sodium salts are preferred. Specific examples of such alkali metal salts include monosodium cytidylate and disodium cytidylate, disodium cytidylate being preferred from the standpoint of handleability.

The concept of uridylic acid as used herein includes salts as described above. The salts of uridylic acid include alkali metal salts such as sodium and potassium salts; alkaline earth metal salts such as calcium, magnesium and barium salts; basic amino acid salts such as arginine and lysine; ammonium salts such as ammonium and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, alkali metal salts such as sodium salts are preferred. Specific examples of such alkali metal salts include monosodium uridylate and disodium uridylate, disodium uridylate being preferred from the standpoint of handleability.

The above active ingredients may be used alone or in combination of two or more.

There is no particular limitation on the origins of the active ingredients, and those derived from natural products such as yeast, bacteria, seafood, animals, and plants are suitable.

It is believed that the BDNF production promoter according to the present invention contributes to improvement of the QOL of patients suffering from diseases caused by BDNF deficiency and/or reduction, or elderly people. Specifically, the promoting of the production of BDNF is expected to achieve improvement of cognitive function and/or memory, improvement of depression, improvement of neurodegenerative diseases, reduction of anxiety, improvement of energy, improvement of sugar and/or lipid metabolism, anti-obesity, and improvement of reproductive function.

In addition, daily intake of the BDNF production promoter according to the present invention is expected to have preventive effects against various symptoms caused by BDNF deficiency, including, specifically, maintenance of cognitive function and/or memory, prevention of depression, prevention of neurodegenerative diseases, suppression of anxiety, maintenance of energy, maintenance of sugar and/or lipid metabolism, prevention of obesity, and maintenance of reproductive function.

The BDNF production promoter according to the present invention can be used for practical purposes as a composition for food and drink products, supplements, prepared milk powder, enteral nutritional supplements, healthy food and drink products (including food for specified health uses and foods with function claims), additives for animal feed, and pharmaceutical products for humans or animals other than humans.

When the BDNF production promoter according to the present invention is provided as the food and drink product, health food and drink product or prepared milk powder, it can be made into a food or drink product having a BDNF production promoting effect by adding the above active ingredients to the known food and drink product as appropriate. The food and drink products of interest include milk and dairy products, seasonings, beverages, confectioneries, breads, noodles, oils and fats, processed meat products, processed marine products, processed agricultural products, frozen foods, and instant foods.

The novel food and drink products that have the BDNF production promoting effect can also be produced by mixing with materials for the food and drink products. The shape of the food and drink products of interest can be selected from various forms, such as tablets, granules, capsules, powders, solutions, syrups, emulsions, and pastes. In addition to the active ingredient according to the present invention, various excipients and seasoning ingredients that can be used as foods may be added as needed in the production of those food products.

The food and drink product as described above may be provided and sold as a food and drink product labeled with the health application for promoting the BDNF production. The act of "labeling" includes all acts to make the above application known to users, and all expressions that may evoke or analogize the above application fall under the act of "labeling" in this invention, regardless of the purpose of the labeling, the content of the labeling, and the object or medium to be labeled.

It is preferable that the above "labeling" be made by means of expressions that enable users to directly recognize the above application. Specific examples include the act of assigning, delivering, displaying for the purpose of assignment or delivery, or importing of goods or packages of the goods in relation to the food and drink products, which describe the above application, or the act of displaying or distributing advertisement materials, price lists or transaction documents in relation to the goods, which describes the above application, and the act of providing information about these contents, which describes the above application, through electromagnetic means (e.g., through the internet).

It is preferable that the contents of the labeling are those approved by the government or the like (e.g., labeling that has been approved based on various systems established by the government and is performed in a manner based on such approval). It is also preferable to attach such labeling to packages, containers, catalogs, pamphlets, POP, and other promotional materials at the places of sales, and other documents.

When the BDNF production promoter according to the present invention is practically provided as a pharmaceutical, supplement, enteral nutritional product, and the like, the above active ingredient can be formulated alone or in combination with formulation aids or the like. The formulations may be orally or parenterally administered, and they are preferably orally or enterally administrated.

The formulations as described above can be in the forms such as tablets, granules, capsules, pills, dispersions, solutions, syrups, and emulsions for oral administration, and injections, sprays, ointments, and patches for parenteral administration.

In addition to the active ingredient according to the present invention, the formulation may use any formulation aid such as excipients, binders, disintegrants, lubricants, taste masking agents, dissolution aids, suspending agents, coating agents, and the like, appropriately in combination, according to each dosage form.

The amount of the above active ingredient formulated in the BDNF production promoter according to the present invention may be appropriately selected from the range of 0.001 to 99.5% (w/w), depending on the purpose of use (prevention, health or symptom relief, etc.), age of the subject, method of administration or ingestion, dosage form, and the like. For example, in the case of tablets, granules, capsules, powders, powder dispersion, and the like, the formulated amount is preferably 0.1 to 99% (w/w), and in the case of solutions, syrups, emulsions, and the like, the formulated amount is preferably 0.002 to 20% (w/w).

The amount of the BDNF production promoter according to the present invention administered or ingested may be appropriately selected from the range of about 0.1 mg to about 800 g per a day, although it will vary depending on the subject's age, weight, the method of administration or ingestion, and the like. Among these, the amount of the BDNF production promoter administered or ingested is preferably 1 mg to 500 g, and more preferably 5 mg to 50 g.

The present invention is based on the findings of a novel attribute that pyrimidine nucleotides or precursors thereof have a clear BDNF production promoting effect, and of suitability for use in novel and highly safe BDNF production promoters in which pyrimidine nucleotides or precursors thereof exhibit a BDNF production promoting effect when they are used as foods, feeds, pharmaceuticals, quasi-drugs, and the like, due to that attribute. Thus, the present invention is exemplified as follows:

[1] A BDNF production promoter comprising at least one pyrimidine nucleotide or a precursor thereof as an active ingredient.
[2] The BDNF production promoter according to [1], wherein the pyrimidine nucleotide or the precursor thereof is one or more selected from the group consisting of cytidylic acid, uridylic acid, cytidine, and uridine.
[3] The BDNF production promoter according to [1] or [2], wherein the BDNF production promoter has two or more effects selected from improvement of cognitive function and/or memory, improvement of depression, improvement of neurodegenerative disease, reduction of anxiety, improvement of energy, improvement of sugar and/or lipid metabolism, anti-obesity, and improvement of reproductive function.
[4] The BDNF production promoter according to [1] or [2], wherein the BDNF production promoter has two or more effects selected from maintenance of cognitive function and/or memory, prevention of depression, prevention of neurodegenerative disease, suppression of anxiety, maintenance of energy, maintenance of sugar and/or lipid metabolism, prevention of obesity, and maintenance of reproductive function.
[5] A method for promoting BDNF production, comprising administering at least one pyrimidine nucleotide or a precursor thereof.
[6] The method according to [5], wherein the pyrimidine nucleotide or the precursor thereof is one or more selected from the group consisting of cytidylic acid, uridylic acid, cytidine, and uridine.
[7] A pyrimidine nucleotide or a precursor thereof for use in promoting BDNF production.
[8] The pyrimidine nucleotide or precursor thereof according to [7], wherein the pyrimidine nucleotide or precursor thereof is one or more selected from the group consisting of cytidylic acid, uridylic acid, cytidine and uridine.
[9] Use of at least one pyrimidine nucleotide or a precursor thereof, for promoting BDNF production.
[10] The use according to [9], wherein the pyrimidine nucleotide or the precursor thereof is one or more selected from the group consisting of cytidylic acid, uridylic acid, cytidine and uridine.

### [Examples]

The present invention will be described by Examples, but the present invention is not limited by these Examples.

### (Example 1) BDNF Gene Expression Promoting Effect-1

Mouse myoblast cell line C2C12 cells (RIKEN BRC, RCB0987) were suspended in a growth medium (DMEM containing 10% FBS and antibiotics), seeded in a 24-well plate, and cultured in an incubator at 37°C and 5% CO₂ until the cell density reached 70-90%. The growth medium was removed and replaced with a differentiation induction medium (DMEM containing 2% horse serum and antibiotics). After culturing it for 6 days while changing the medium once every 2 days, cytidylic acid (CMP) or uridylic acid (UMP) was added to a final concentration of 1 mM and cultured for 4 hours. The medium was removed, and the total RNA was extracted from the cells using NucleoSpin^{®} RNA (TAKARA BIO INC) .

Using the total RNA as a template, a reverse transcription reaction solution was prepared using ReverTra Ace^{®} qPCR RT Kit (TOYOBO). The reverse transcription reaction solution, GoTaq qPCR Master Mix (Promega), and a real-time PCR device Thermal Cycler Dice^{®} Real Time System (TAKARA BIO INC) were used to measure amounts of mRNAs of BDNF and β-actin as an internal standard. Analysis was performed by relative quantification, and the amount of the mRNA was corrected using the amount of mRNA of β-actin as an endogenous control.

For statistical analysis, Dunnett's multiple comparison test was conducted using, as a control, a differentiation inducing medium-treated group that did not contain the test substance. The threshold of statistical significance was set at 5%. In addition, unless otherwise specified, including the following Examples, each sample was examined at n = 3.

The results of Example 1 are shown in FIG. 1.

As shown in FIG. 1, the amount of mRNA of BDNF in C2C12 cell-derived myotube cells was significantly increased by treatment with cytidylic acid (CMP) or uridylic acid (UMP).

The results of Example 1 demonstrated that the production of BDNF in muscle was promoted by the pyrimidine nucleotide or precursor thereof. That is, this Example demonstrated that the pyrimidine nucleotides or precursors thereof were useful as the BDNF production promoter.

### (Example 2) BDNF Gene Expression Promoting Effect-2

Rat adrenal medulla brown tumor-derived cells (PC-12 cells, JCRB0733), used as nervous system model cells, were suspended in DMEM containing 20% FBS and antibiotics, seeded on a collagen-coated 24-well plate at a density of 2.4 x 10⁵ cells/well, and cultured in an incubator at 37°C and 5% CO₂ for 18 hours. Uridylic acid (UMP) was added to a final concentration of 500 µM, and cultured for 4 hours.

Subsequently, mRNA was extracted and an amount of mRNA of BDNF was evaluated by reverse transcription and real-time PCR analysis in the same procedure as that of Example 1. The results of Example 2 are shown in FIG. 2.

As shown in FIG. 2, the amount of mRNA of BDNF in PC-12 cells was significantly increased by treatment with uridylic acid (UMP).

The results of Example 2 revealed that the BDNF production promoting effect of the pyrimidine nucleotide was exerted not only in muscles but also in nerves, indicating that the pyrimidine nucleotide or precursor thereof promoted BDNF production in tissues other than muscles as well.

### (Example 3) BDNF Gene Expression Promoting Effect-3

PC-12 cells were suspended in DMEM containing 5% FBS (fetal bovine serum), 10% horse serum, and antibiotics (100 units/ml of penicillin, 100 ug/ml of streptomycin), seeded on a collagen-coated 24-well plate at a density of 2.4 x 10⁵ cells/well, and cultured in an incubator at 37°C and 5% CO₂ for 18 hours. Uridine, a precursor of uridylic acid, was added to a final concentration of 200 µM, and cultured for 1 hour.

Subsequently, mRNA was extracted, and the amount of mRNA of BDNF was evaluated by reverse transcription and real-time PCR analysis in the same procedure as that of Example 1.

The results of Example 3 are shown in FIG. 3.

As shown in FIG. 3, the amount of mRNA of BDNF in PC-12 cells was significantly increased by treatment with uridine.

The results of Example 3 demonstrated that the nucleoside, a precursor of the pyrimidine nucleotide, had the effect of promoting BDNF production.

The results of Examples 1, 2, and 3 revealed that uridylic acid, cytidylic acid, and uridine had the effect of increasing BDNF mRNA. Also, the results revealed that they had the effect of increasing BDNF mRNA in both C2C12 cell-derived myotube cells, which are widely used as a muscle model, and PC-12 cells, which are widely used as a nerve model. In summary of the above results, pyrimidine nucleotides or their precursors are considered to promote the production of BDNF in tissues throughout the body, including at least muscles and nerves, and pyrimidine nucleotides or their precursors would be very useful as BDNF production promoters.

### (Example 4) BDNF Gene Expression Promoting Effect-4

PC-12 cells were suspended in DMEM containing 20% FBS and antibiotics, seeded on a collagen-coated 24-well plate at a density of 2.4 x 10⁵ cells/well, and cultured in an incubator at 37°C and 5% CO₂ for 18 hours. Uridine was added to a final concentration of 200 µM, and cultured for 24 hours.

The medium was removed by suction, and 100 µl of RIPA buffer (50 mM Tris-HCl (pH 7.6), 150 mM NaCl, 1% NP-40, 0.5% sodium deoxycholate, 1% SDS) containing 1% protease inhibitor cocktail (nacalai tesque) was added, and the cells were disrupted by pipetting and ultrasonication. The amount of BDNF in the cell lysate was measured using a Rat BDNF ELISA Kit (RayBiotech). The amount of protein in the cell lysate was measured using a Micro BCA^{™} Protein Assay Kit (Thermo Fisher Scientific), and the amount of BDNF per total protein mass was calculated.

For statistical analysis, a t-test was conducted using, as a control, a differentiation inducing medium-treated group that did not contain the test substance. The threshold for statistical significance was set at 5%.

The results of Example 4 are shown in FIG. 4.

As shown in FIG. 4, the amount of BDNF per total protein mass in PC-12 cells was significantly increased by treatment with uridine.

The results of Example 4 demonstrated that uridine has the effect of increasing the amount of BDNF protein mass.

The results of Example 4 confirmed that pyrimidine nucleotides or precursors thereof increased the amount of a protein mass of BDNF through an increase in the amount of mRNA of BDNF.

## Claims

1. A BDNF production promoter comprising at least one pyrimidine nucleotide or a precursor thereof as an active ingredient.

2. The BDNF production promoter according to claim 1, wherein the pyrimidine nucleotide or the precursor thereof is one or more selected from the group consisting of cytidylic acid, uridylic acid, cytidine, and uridine.

3. The BDNF production promoter according to claim 1 or 2, wherein the BDNF production promoter has two or more effects selected from improvement of cognitive function and/or memory, improvement of depression, improvement of neurodegenerative disease, reduction of anxiety, improvement of energy, improvement of sugar and/or lipid metabolism, anti-obesity, and improvement of reproductive function.

4. The BDNF production promoter according to claim 1 or 2, wherein the BDNF production promoter has two or more effects selected from maintenance of cognitive function and/or memory, prevention of depression, prevention of neurodegenerative disease, suppression of anxiety, maintenance of energy, maintenance of sugar and/or lipid metabolism, prevention of obesity, and maintenance of reproductive function.

5. A method for promoting BDNF production, comprising administering at least one pyrimidine nucleotide or a precursor thereof.

6. The method according to claim 5, wherein the pyrimidine nucleotide or the precursor thereof is one or more selected from the group consisting of cytidylic acid, uridylic acid, cytidine, and uridine.

7. A pyrimidine nucleotide or a precursor thereof for use in promoting BDNF production.

8. The pyrimidine nucleotide or precursor thereof according to claim 7, wherein the pyrimidine nucleotide or precursor thereof is one or more selected from the group consisting of cytidylic acid, uridylic acid, cytidine and uridine.

9. Use of at least one pyrimidine nucleotide or a precursor thereof, for promoting BDNF production.

10. The use according to claim 9, wherein the pyrimidine nucleotide or the precursor thereof is one or more selected from the group consisting of cytidylic acid, uridylic acid, cytidine and uridine.
